# EUROPÄISCHE PATENTSCHRIFT

(11) **EP 1 296 626 B1**
(45) Veröffentlichungstag und Bekanntmachung des Hinweises auf die Patenterteilung: **18.10.2006**
(21) Anmeldenummer: 01935908.2
(22) Anmeldetag: 11.06.2001
(51) Int. Cl.: A61F 15/00, A61F 13/84

(54) **VORRICHTUNG ZUR AUFBEWAHRUNG UND ABGABE VON WINDELN**
DEVICE FOR STORING AND DISPENSING DIAPERS
DISPOSITIF DE STOCKAGE ET DE DISTRIBUTION DE COUCHES

(30) Priorität: 11.06.2000 CH 115500
(43) Veröffentlichungstag der Anmeldung: 02.04.2003
(73) Patentinhaber: Yuvayapar, Sibylle, 5323 Rietheim (CH)
(72) Erfinder: Yuvayapar, Sibylle, 5323 Rietheim (CH)
(74) Vertreter: Spierenburg, Pieter
(86) Internationale Anmeldenummer: PCT/CH2001/000362
(87) Internationale Veröffentlichungsnummer: WO 2001/095846

(56) Entgegenhaltungen:
- AU-B- 540 200
- DE-U- 29 607 763
- DE-U- 29 722 873
- DE-U- 29 905 377
- FR-A- 2 773 315
- GB-A- 969 871
- US-A- 2 700 485
- US-A- 3 744 866
- US-A- 4 365 709
- US-A- 4 577 773
- US-A- 5 163 581

## Beschreibung

Die Erfindung betrifft eine Vorrichtung zur Aufbewahrung und Abgabe von Windeln nach dem Oberbegriff des Patentanspruchs 1.

Wegwerfwindeln für Babys oder Kleinkinder sind allgemein bekannt und sind in grösseren Packungen von 30 bis 40 Stück auf dem Markt erhältlich. In der Regel werden die Packungen aus einem dünnen Plastikmaterial zum Gebrauch aufgerissen und die einzelnen Wegwerfwindeln daraus entnommen. Da die üblichen Wickeltische wenig Platz bieten, stellt man die Packungen auf den Boden, was beim Wickeln des Babys oder des Kleinkindes nicht ungefährlich sein kann. Das Baby ist nämlich während kurzer Zeit unbeobachtet, was bei einem lebhaften Baby sehr leicht zu Unfällen führen kann. Wenn zusätzlich Feuchttücher und andere Utensilien für die Pflege des Babys oder Kleinkindes benötigt werden, steigt die Unfallgefahr noch weiter. Bei Zwillingen sind bald zwei Personen nötig, um Unfälle beim Wickeln zu verhindern.

Die Windeln könnte man natürlich in einem Oberschrank versorgen und diese beim Wickeln daraus entnehmen. Wegen der schlechten Stapelbarkeit der Windeln ist diese Lösung aber auch nicht sehr befriedigend. Denn nicht selten fällt der Stapel um und müssen die Windeln wieder gesammelt werden, mit denselben Risiken wie oben.

Aus FR-A-2 773 315 ist nun einer Behälter zur Aufbewahrung von Wegwerfwindeln bekannt, der eine Bodenplatte, darauf befestigte Seitenwände und eine Rückwand und einen kippbaren Vorderteil aufweist. Der Vorderteil wird mit einem Verriegelungshaken geschlossen. Auf den Innenseiten der Seitenwände sind parallele Gleitbahnen vorgesehen, in welche eine Platte hineingeschoben werden kann, um die Grösse des Behälters an die Windelgrösse anzupassen. Oben ist ein schwenkbarer Deckel vorgesehen. Der kippbare Vorderteil weist unten eine Entnahmeöffnung für eine einzelne Windel auf. Ferner ist im unteren Bereich des Vorderteils ein kleiner Längsschlitz vorgesehen, um den Füllungsgrad zu erkennen.

Dieser bekannte Behälter hat den Nachteil, dass die Bodenplatte wesentlich grösser als die Zarge des Behälters ist. Für das Befüllen des Behälters müssen sowohl der Vorderteil als auch der Deckel aufgeklappt werden (vergleiche Fig. II). Je nachdem wie hoch die Zarge ist, wird das Auffüllen problematisch, weil man die Windeln einzeln tief in den Behälter legen muss. Ein ganzer Stapel kann kaum richtig eingefüllt werden. Dies ist besonders problematisch, bei kleineren Windelgrössen.

Der vorliegenden Erfindung liegt nun die Aufgabe zugrunde, eine Vorrichtung für die Aufbewahrung und Abgabe von Windeln zu schaffen, die ein leichtes Auffüllen von Wegwerfwindeln und eine besonders einfache Entnahme einer einzelnen Windel ermöglicht.

Diese Aufgabe wird durch eine Vorrichtung mit den Merkmalen des Patentanspruchs 1 gelöst.

Die erfindungsgemässe Vorrichtung hat den wesentlichen Vorteil, dass eine einzelne Wegwerfwindel problemlos entnommen werden kann, ohne das zu wickelnde Baby oder Kleinkind aus den Augen zu verlieren. Wenn die Vorrichtung auf der richtigen Höhe angeordnet ist, kann man mit der einen Hand eine Windel fassen und mit der anderen Hand das Baby festhalten. Dadurch wird das Unfallrisiko äusserst gering. Ferner bietet die Vorrichtung auch eine einfachere Handhabung und dadurch auch eine Zeitersparnis. Da die Vorrichtung auf die Grösse der gängigen Wegwerfwindeln angepasst ist, wird auch Platz gespart, denn die Grösse der normalen Schränke erlaubt keine optimale Befüllung.

Weitere Vorteile der Erfindung folgen aus den abhängigen Patentansprüchen und aus der nachfolgenden Beschreibung, in welcher die Erfindung anhand eines in den schematischen Zeichnungen dargestellten Ausführungsbeispieles näher erläutert wird. Es zeigt:
- Fig. 1: eine erste Ausführungsform eines Windelspenders,
- Fig. 2: eine zweite Ausführungsform des Windelspenders,
- Fig. 3: das unterhalb des Windelspenders der Fig. 2 angeordnete Behältnis für Feuchttücher,
- Fig. 4: eine dritte Ausführungsform des Windelspenders,
- Fig. 5: eine vierte Ausführungsform des Windelspenders,
- Fig. 6: eine fünfte Ausführungsform des Windelspenders,
- Fig. 7: eine sechste Ausführungsform des Windelspenders,
- Fig. 8: eine siebte Ausführungsform des Windelspenders,
- Fig. 9: eine achte Ausführungsform des Windelspenders,
- Fig. 10: eine neunte Ausführungsform des Windelspenders,
- Fig. 11: eine erste Variante der verschiebbaren Trennwand,
- Fig. 12: eine zweite Variante der verschiebbaren Trennwand,
- Fig. 13: eine dritte Variante der verschiebbaren Trennwand,
- Fig. 14: eine vierte Variante der verschiebbaren Trennwand,
- Fig. 15: eine fünfte Variante der verschiebbaren Trennwand, und
- Fig. 16 und 17: zwei Ausführungen von ankoppelbaren Behältern für Feuchttücher.

In den Figuren sind für dieselben Elemente jeweils dieselben Bezugszeichen verwendet worden und erstmalige Erklärungen betreffen alle Figuren, wenn nicht ausdrücklich anders erwähnt.

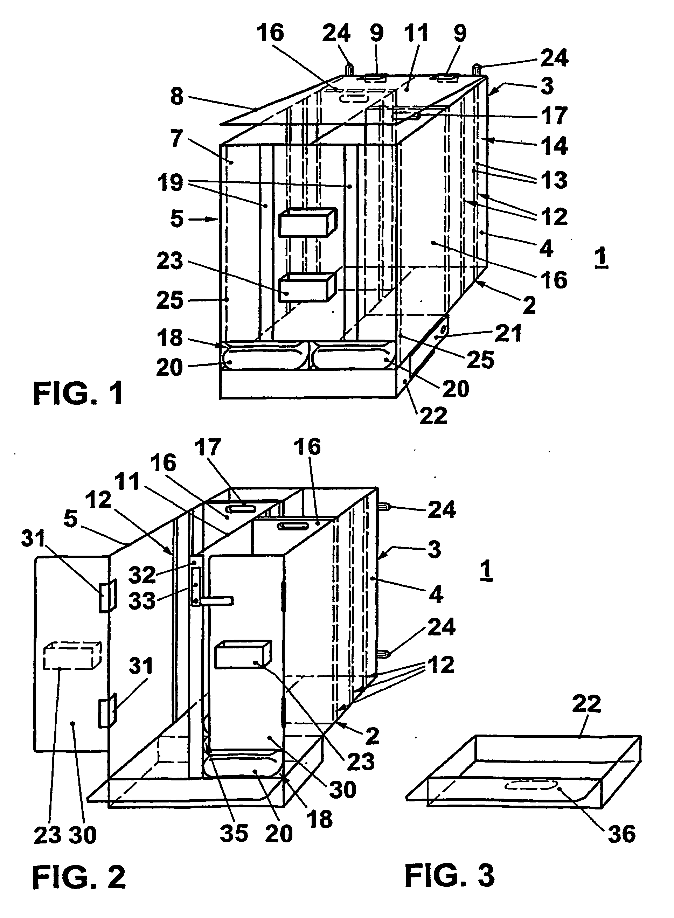

In der Figur 1 ist ein Windelspender 1 schematisch dargestellt, der aus einer Bodenplatte 2, einer Rückwand 3, zwei aufstehenden, sich gegenüber stehenden Seitenwänden 4 und 5 und einer Vorderwand 7 besteht und so ein quaderförmiges Behältnis bildet. An der Rückwand 3 ist ein Deckel 8 mittels Scharnieren 9 aufklappbar befestigt. Wie ersichtlich ist parallel zu den beiden Seitenwänden 4 und 5 eine Zwischenwand 11 vorgesehen, die auf der Bodenplatte 2 und an der Rückwand 3 befestigt ist. Auf den Innenseiten der beiden sich gegenüberliegenden Seitenwänden 4 und 5 und beidseitig auf der Zwischenwand 11 sind parallele Längsführungen 12 vorgesehen, die aus zwei parallel zu einander angeordnete Stegen 13 bestehen, die eine Nut 14 freilassen. Die Längsführungen 12 liegen einander derart gegenüber, dass eine versetzbare Trennwand 16 in die jeweiligen Nuten 14 eingeschoben werden kann, derart dass sie parallel zur Rückwand 3 verläuft. So kann man durch richtiges Einschieben der Trennwand 16 die Länge der Aufnahme auf die Windelgrösse anpassen. Oben an den Trennwänden 16 kann ein Handgriff 17 in der Form eines nierenförmigen Ausschnittes vorgesehen sein, damit sie leichter herausgezogen werden können. Die Vorderwand 7 ist nun in der Länge weniger gross als die Rückwand 3, so dass sie im unteren Bereich des Behältnisses einen Spalt 18 freilässt, der etwas höher ist als die Höhe einer Wegwerfwindel 20. Die Vorderwand 7 ist mit streifenförmigen, in Längsrichtung verlaufenden Sichtfenstern 19 versehen, so dass man die Befüllung des Windelspenders 1 mit Windeln 20, insbesondere Wegwerfwindeln, einfach überprüfen kann. Diese Vorderwand 7 ist nun in zwei Längsnuten 25 in den Seitenwänden 4 und 5 verschiebbar. Die beiden Längsnuten 25 reichen dabei nur bis zur Oberseite des Spaltes 18. Damit lässt sich die Vorderwand 7 einfach wegschieben, so dass die gesamte Vorderseite des Windelspenders 1 freigegeben wird, und dieser mit einem Stapel Windeln 20 äusserst einfach befüllt werden kann. Der Abstand zwischen den Seitenwänden 4 und 5 und der Zwischenwand 11 sollte ferner im wesentlichen der Breite der grösstmöglichen Windel 20 entsprechen. Da die Breiten der verschiedenen Windelgrössen wesentlich weniger variieren als deren Längen, werden die eingefüllten Windeln 20 optimal von allen Seiten gestützt. Unterhalb des Windelspenders 1 ist eine mit einem Schieber 21 verschliessbare Schachtel 22 vorgesehen, in welcher Feuchttücher oder dergleichen aufbewahrt werden können. Auf der Vorderseite der Vorderwand 7 sind noch zwei Ablagefächer 23 vorgesehen, die für Wattenstäbchen, Tuben und dergleichen vorgesehen sind. Ferner sind auf der Rückwand 3 noch zwei als Oesen ausgebildete Halterungen 24 vorgesehen, damit der Windelspender 1 an die Wand aufgehängt werden kann.

In Figur 2 ist eine Variante des Windelspenders 1 dargestellt, wobei die Oberseite offengelassen ist und auf der Vorderseite zwei Klapptüren 30 vorgesehen sind, die mittels Scharnieren 31 an den Seitenwänden 4 und 5 drehbar befestigt sind. An der Vorderkante der Trennwand 11 ist ein Quersteg 32 vorgesehen, der zwei drehbare Riegel 33 aufweist, um die Klapptüren 30 zu verschliessen. Wie ersichtlich verbleibt zwischen der geschlossenen Klapptüre 30 und dem Quersteg 32 ein offener Längsspalt 35, der als Sichtfenster für die Befüllung des Windelspenders 1 mit Windeln 20 dient. Unterhalb des Windelspenders 1 ist eine Schachtel 22 vorgesehen, die hier auf der Vorderseite einen Klappdeckel 36 aufweist. Selbstverständlich kann auch eine Art Schublade vorgesehen sein, um Feuchttücher oder dergleichen aufzubewahren. Oben kann zusätzlich eine (hier nicht dargestellte) Deckplatte vorgesehen sein.

Es versteht sich für den Fachmann, dass die Trennwände 16 auch auf eine andere Art verstellbar mit den Seitenwänden 4 und 5 und der Zwischenwand 11 befestigt werden können. Beispielsweise könnten beidseits an den Längsrändern der Trennwände 16 Streifen aus einem Klettverschluss vorgesehen sein, die mit übereinstimmenden Gegenstreifen auf den Seitenwänden 3 und 4 und auf der Zwischenwand 11 genau so eine Verstellung der Trennwände 16 ermöglichen.

Die dargestellten Windelspender 1 lassen sich einstückig in einem Spritzgussverfahren aus einem geeigneten Kunststoffmaterial wie beispielsweise Polypropylen oder dergleichen herstellen. Die Dicke der Seitenwände 4 und 5 kann dabei so gewählt sein, dass lediglich Nuten 14 in den Seitenwänden 4 und 5 vorgesehen sind, was bei der Befüllung mit Wegwerfwindeln günstiger sein kann, da keine vorstehende Teile mehr vorhanden sind.

In einer besonders einfachen Ausführungsform kann auch auf die Zwischenwand 11 verzichtet werden und eine einzige Trennwand 16 vorgesehen sein, um eine Anpassung auf die Windelgrösse vorzunehmen.

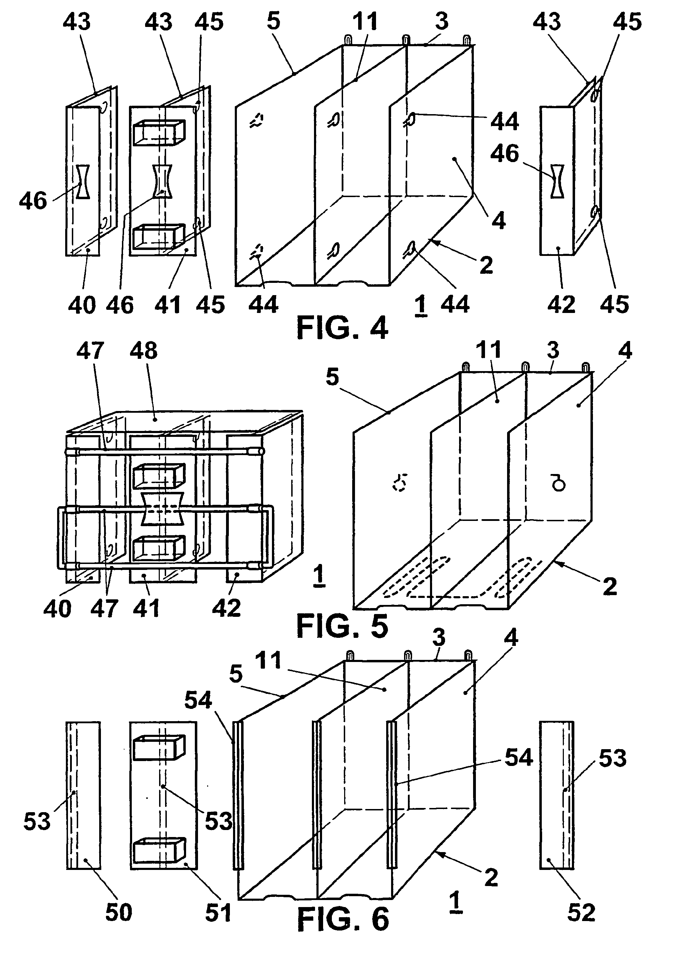

In den Figuren 4 bis 6 sind nun drei weitere Ausführungsformen des obigen Windelspenders 1 dargestellt. Gemäss Figur 4 kann die Vorderwand 7 aus drei einzelnen, winkelförmigen Aufsteckteilen 40, 41 und 42 bestehen, die je einen doppelwandigen Aufsteckbereich 43 aufweisen. Die Seitenwände 4 und 5 und die Zwischenwand 11 weisen ausgestanzte Zungen 44 auf, die in entsprechende Löcher 45 der Aufsteckteile 40, 41 und 42 eingreifen können. Zum Lösen werden die Zungen 44 eingedrückt und die Aufsteckteile 40, 41 und 42 weggenommen. In einer Variante gemäss Figur 5 sind die Aufsteckteil 40, 41 und 42 mit Querstäben 47 miteinander verbunden und so gemeinsam aufsteckbar. Oben auf den Aufsteckteilen 40, 41 und 42 ist ferner eine gemeinsame Abdeckung 48 als weitere Verbindung vorgesehen. Eine weitere Variante ist in Figur 6 dargestellt, wobei die Aufsteckteile 50, 51 und 52 mittels Klemmteile 53 auf Längsschienen 54 geschoben werden können. Mit einem nicht dargestellten Anschlag wird die richtige Höhe der Entnahmeöffnung 18 bestimmt.

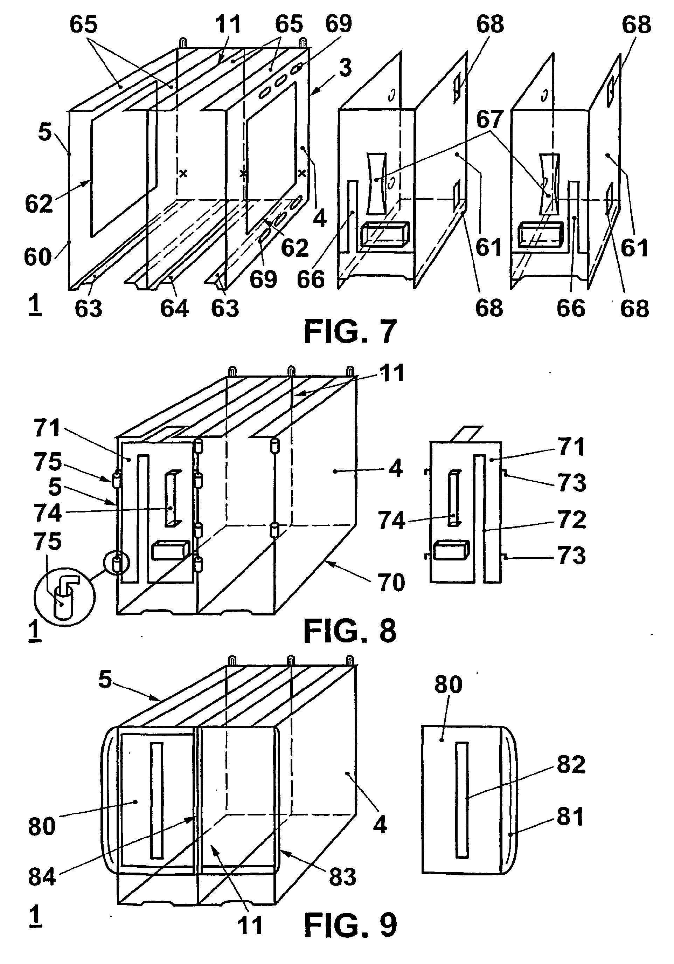

In Figur 7 ist eine weitere Variante dargestellt, bei welcher der Windelspender 1 aus einem Aufnahmeteil 60 und einem Einschubteil 61 besteht. Der Aufnahmeteil 60 weist Seitenwände 4 und 5, eine Zwischenwand 11 und eine Rückwand 3 auf. Die Seitenwände 4 und 5 weisen grosse vierkante Ausschnitte 62 auf. Unten an den Seitenwänden sind Laufschienen 63 und an der Zwischenwand 11 ist eine Doppelschiene 64 vorgesehen, auf welchen die Einschubteile 61 in den Aufnahmeteil 60 hineingeschoben werden können. Oben sind streifenförmige Abdeckungen 65 vorgesehen, die die Höhe des Windelstapels begrenzen. Die Einschubteile 61 sind als Schubfächer mit einem viereckigen, länglichen Ausschnitt als Sichtfenster 66 und einem Handgriff 67 ausgebildet. Ferner sind vorstehende Zungen 68 vorgesehen, die in entsprechende Löcher 69 des Aufnahmeteils 60 einschnappen. Es sind nun mehrere, hintereinander liegende Löcher 69 vorgesehen, so dass der Windelspender 1 auf die Grösse der eingefüllten Windeln anpassbar ist. Die Länge des Aufnahmeteiles 60 und diejenige des Einschubteiles 61 entsprechen der Länge der kleinsten einzufüllenden Windeln (hinterste Position). Wenn grössere Windeln eingefüllt werden, steht der Einschubteil 61 ein Stück weit über dem Aufnahmeteil 60 vor.

Eine Variante des Windelspenders 1 gemäss Figur 7 ist in der Figur 8 dargestellt, wobei hier eine Bodenplatte 70 und anstelle von Einschubteilen 61 eine Abdeckplatte 71 mit einem schlitzförmigen Sichtfenster 72, Haken 73 und einen Handgriff 74 vorgesehen sind. Die Haken 73 greifen in entsprechende Ösen 75 an den Seitenwänden 4 und 5 und an der Zwischenwand 11 ein, so dass auch hier wieder eine Einstellung auf die Windelgrösse möglich ist.

In Figur 9 ist eine weitere Variante des Windelspenders 1 dargestellt, wobei hier als Abdeckung ein Schiebeelement oder Schieber 80 mit seitlichem Handgriff 81 und einem länglichen Sichtfenster 82 vorgesehen ist. Der Schieber 80 ist in einen Schlitz 83 in den Seitenwänden 4 und 5 hineinschiebbar. Zur Stabilität sind auf der Zwischenwand 11 entsprechende Längsnuten 84 vorgesehen, in welche die Enden der Schieber 80 festgeklemmt werden. Wenn mehrere parallele Schlitze 83 in den Seitenwänden 4 und 5 und entsprechende gegenüberliegende Längsnuten 84 vorgesehen sind, kann man auf diese Weise die Grösse des Behältnisses auf die Windelgrösse anpassen.

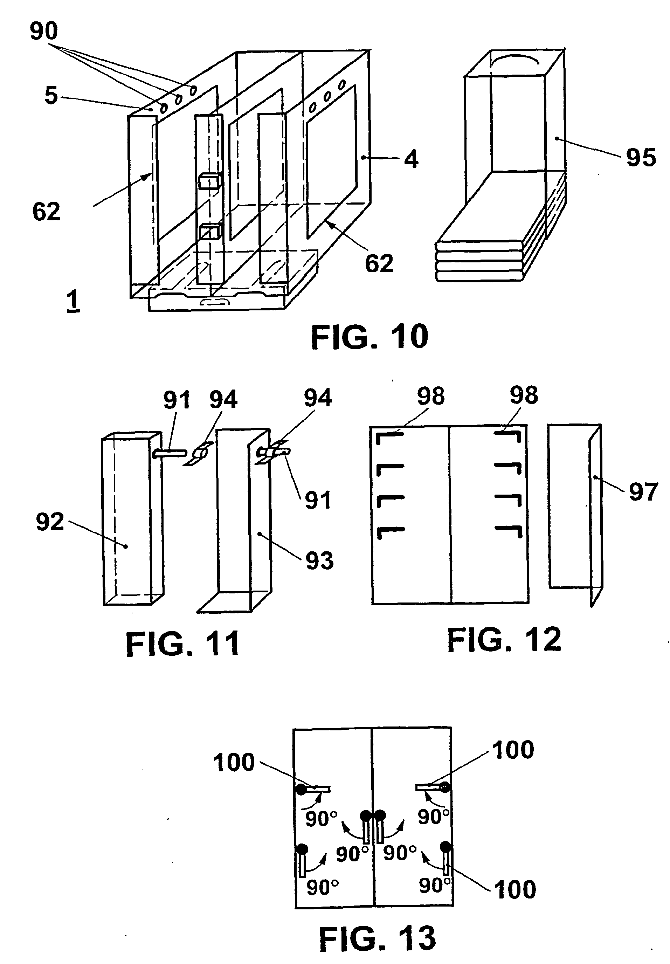

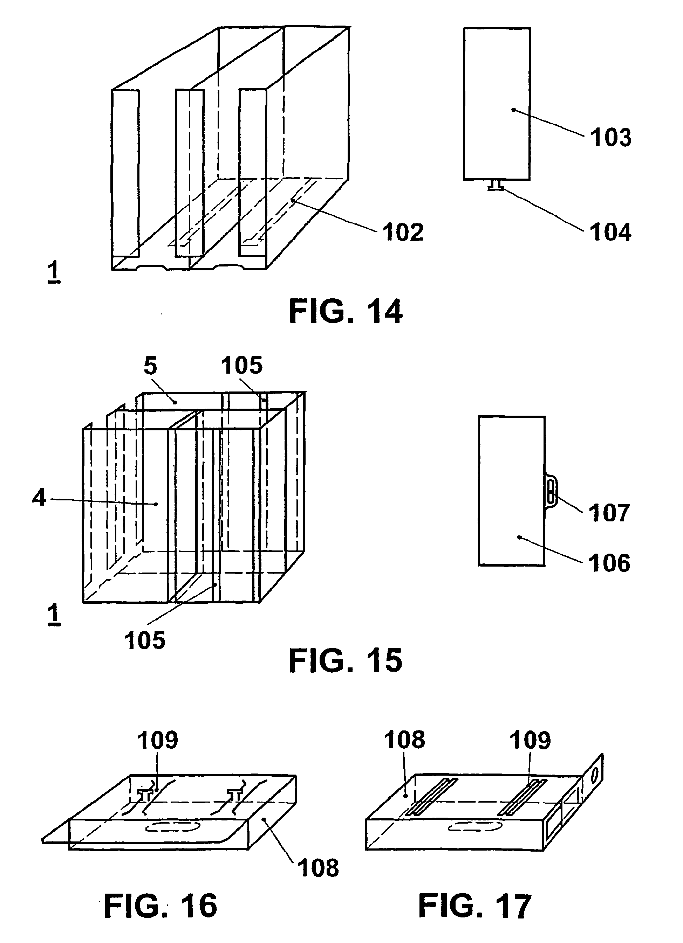

Eine weitere Variante des Windelspenders 1 ist in Figur 10 dargestellt. Die Vorderseite ist ähnlich wie die erste Ausführung der Figur 1 ausgebildet. Hier sind die Seitenwände 4 und 5 mit grossen vierkanten Auschnitten 62 wie in Figur 7 ausgebildet. Oberhalb sind Bohrungen 90 vorgesehen, in welche Schraubstifte 91 der in Figur 11 dargestellten Trennwände 92 oder 93 hineingesteckt und mit Muttern 94 befestigt werden können. Auch kann eine hochkant aufgestellte Schublade 95 verwendet werden, die mittels hier nicht dargestellten Stiften, die in den Bohrungen 90 passen, an der richtigen Stelle platziert sind. Gemäss Figur 12 können auch winkelförmige Trennwände 97 vorgesehen sein, die in entsprechend winkelförmige Schlitze 98 im Bodenbereich des Windelspenders 1 eingesteckt werden. Eine andere Möglichkeit nach Figur 13 besteht darin, dass wechselseitig Scharnierteile 100 vorgesehen sind, die um 90° zu einem Anschlag aufgeklappt werden können, um die richtige Windelgrösse festzuhalten. Gemäss der Ausführung der Figur 14 ist ein sacklochartiger Schlitz 102 im Boden des Windelspenders 1 vorgesehen, in welcher eine Trennwand 103 mit einem Schieber 104 schiebbar ist. Dazu können entsprechende sägezahnförmige Rillen vorgesehen sein, um die Trennwand 103 an der gewünschten Stelle festzuhalten. Die Ausführung der Figur 15 betrifft seitlich an den Wänden 4 und 5 angebrachte Schlitze 105, in welche die Trennwände 106 als Schieber mit einem daran angebrachten Griff 107 hineinschiebbar sind.

In den Figuren 16 und 17 sind noch zwei Ausführungen von Behältern 108 für Feuchttücher dargestellt, die mittels Schienen 109 an der Unterseite des Windelspenders angekoppelt werden können (vergleiche Figur 5).

Die nun hier dargestellten Ausführungen des Windelspenders 1 mit zwei getrennten Kammern für verschieden grosse Windeln eignet sich besonders für Familien mit Kindern verschiedenen Alters. Solche Spender sind aber auch sehr geeignet für Arztpraxen, Kinderspitäler, Kindergärten und dergleichen.

## Patentansprüche

1. Vorrichtung zur Aufbewahrung und Abgabe von Windeln (20), insbesondere Wegwerfwindeln, die als quaderförmiges Behältnis von einer Bodenplatte (2), Seitenwänden (4, 5) und einer Rückwand (3) ausgebildet und dessen Vorderseite mit einer entfernbaren Abdeckung versehen ist, welche es erlaubt, die Vorderseite mindestens teilweise freizugeben, und die eine im Bereich der Bodenplatte (2) vorgesehene Entnahmeöffnung (18) aufweist, **dadurch gekennzeichnet, dass** die Abdeckung von mindestens einer an der Seitenwand (4, 5) seitlich drehbar befestigten Türe (30) oder von einer durch Schieben entfernbaren Vorderwand (7) ausgebildet ist, um die Vorderseite des quaderförmigen Behältnisses beim Aufklappen der Türe (30) oder Entfernen der Vorderwand (7) vollständig freizugeben, so dass das geöffnete Behältnis mit einem Stapel Windeln von vorne befüllbar ist.

2. Vorrichtung nach Anspruch 1, **dadurch gekennzeichnet, dass** Mittel zum Öffnen des Behältnisses vorgesehen sind, welche von Scharnieren (31), von zwei Längsnuten (25) in den Seitenwänden (4) oder von Aufsteckbereichen (43) an der aus Aufsteckteilen (40, 41, 42) bestehenden Vorderwand (7) gebildet sind.

3. Vorrichtung nach Anspruch 1 oder 2, **dadurch gekennzeichnet, dass** die Entnahmeöffnung (18) im unteren Bereich der Abdeckung (7; 30) vorgesehen ist und etwas höher als die Höhe einer gestapelten Windel (20) ausgebildet ist.

4. Vorrichtung nach Anspruch 3, **dadurch gekennzeichnet, dass** die Abdeckung (7; 30) mit einem Sichtspalt oder einem Sichtfenster versehen ist, um den Füllgrad des Behältnisses festzustellen.

5. Vorrichtung nach Anspruch 4, **dadurch gekennzeichnet, dass** die Breite der als Tore ausgebildete Abdeckung (30) geringer als die Breite der Vorderseite ist, so dass ein offener Längsspalt als Sichtspalt (35) zwischen der Türe (30) und einer Seitenwand (4, 5; 11) verbleibt.

6. Vorrichtung nach einem der Anspruche 1 bis 5, **dadurch gekennzeichnet, dass** Mittel zur Anpassung an die Grösse der Windeln vorgesehen sind.

7. Vorrichtung nach Anspruch 6, **dadurch gekennzeichnet, dass** die Mittel von einer, in parallel zur Rückwand (3) in den Seitenwänden (4, 5) angeordneten Führungen (12) verstellbaren Trennwand (16) gebildet ist

8. Vorrichtung nach Anspruch 7, **dadurch gekennzeichnet, dass** eine Zwischenwand (11) zwischen den beiden Seitenwänden (4, 5) angeordnet ist, und beidseitig an der Zwischenwand (11) Führungen (12) vorgesehen sind, die mit den Führungen (12) an den Seitenwänden (4, 5) übereinstimmen, und zum Einschieben von parallel zur Rückwand angeordneten Trennwänden (16) dienen.

9. Vorrichtung nach Anspruch 1, **dadurch gekennzeichnet, dass** sie zweiteilig mit einem, die offene Vorderseite aufweisenden Aufnahmeteil (60) und einem, die entfernbare Abdeckung aufweisenden Einschubteil (61) ausgebildet ist.

10. Vorrichtung nach Anspruch 9, **dadurch gekennzeichnet, dass** der Einschubteil (61) in verschiedenen, den Windelgrössen entsprechenden Positionen bezüglich des Aufnahmeteils (6) verrastbar ist.

11. Vorrichtung nach Anspruch 1, **dadurch gekennzeichnet, dass** die entfembare Abdeckung von einer aufster-kbaren oder einschiebbaren Abdeckplatte (40, 41, 42; 50, 51, 52; 71) ausgebildet ist.

12. Vorrichtung nach Anspruch 11, **dadurch gekennzeichnet, dass** die Abdeckplatte aus winkelförmigen Aufsteckelementen (40, 41, 42) besteht

13. Vorrichtung nach Anspruch 1, **dadurch gekennzeichnet, dass** die Abdeckung von einem Schiebeelement (80) gebildet ist, das in parallel zur Rückwand (3) in den Seitenwänden (4, 5) angeordneten Schlitzen (83) in vorbestimmten Positionen verstellbar ist.

14. Vorrichtung nach Anspruch 11, **dadurch gekennzeichnet, dass** die Abdeckplatte (71) mittels Haken (73) und Ösen (75) zwischen den Seitenwänden (4, 5) parallel zur Rückwand (3) in vorbestimmten Positionen verstellbar ist.

## Claims

1. Apparatus for storing and dispensing diapers (20), in particular disposable diapers, formed as cuboid recipient from a base plate (2), sidewalls (4, 5) and a back wall (3), and the front side thereof is provided with a displaceable cover, which is adapted to release the front side at least partially, and which comprises a delivery opening (18) in the region of the base plate (2), **characterized in that** the cover is provided at least by a door (30) mounted swingably to one of the sidewalls (4, 4), or by a front wall (7) removable by shifting, in order to release completely the front side of the cuboid recipient at swinging of the door (30) or at shifting of the front wall (7), so that the opened recipient can be replenished frontally with a stack of diapers.

2. Apparatus according to claim 1, **characterized in that** means for opening the recipient are provided, which are formed by hinges (31), by two longitudinal grooves (25) in the side walls (4, 5), or by snap-on zones (43) at the front wall (7) having snap-on elements (40, 41, 42).

3. Apparatus according to claim 1 or 2, **characterized in that** the delivery opening (18) is provided in the lower region of the cover (7; 30) and is formed slightly higher than the height of a stacked diaper (20).

4. Apparatus according to claim 3, **characterized in that** the cover (7; 30) is provided with a gap (35) for visual inspection or with an inspection window, in order to check the filling level of the recipient.

5. Apparatus according to claim 4, **characterized in that** the width of the cover (30) provided as a door is less than the width of the front side, so that an open longitudinally extending gap (35) remains between the door (30) and one of the sidewalls (4, 5; 11).

6. Apparatus according to one of claims 1 to 5, **characterized in that** means are provided for adaptation to the size of the diapers.

7. Apparatus according to claim 6, **characterized in that** the means are formed by a separating wall (16) adjustable in guides (12) arranged in the sidewalls (4, 5) parallel to the back wall (3).

8. Apparatus according to claim 7, **characterized in that** an intermediate wall (11) is arranged between the two sidewalls (4, 5), and that on either side of the intermediate wall (11) guides (12) are provided coordinated with the guides (12) on the sidewalls (4, 5), which serve for taking up separating walls (16) inserted parallel to the back wall (3).

9. Apparatus according to claim 1, **characterized in that** the apparatus is formed in two parts from a take-up part (60) with an open front side and from an insert part (61) with a detachable cover.

10. Apparatus according to claim 9, **characterized in that** the insert part (61) is adapted to be fixed in different positions relative to the take-up part (60) corresponding to the sizes of the diapers in use.

11. Apparatus according to claim 1, **characterized in that** the detachable cover is formed by a snap-on or slide-in cover plate (40, 41, 42; 50, 51, 52; 71).

12. Apparatus according to claim 11, **characterized in that** the cover plate comprises angled snap-on elements (40, 41, 41).

13. Apparatus according to claim 1, **characterized in that** the cover plate is formed by a slide-in element (80), which is adjustable in predetermined positions in slots (83) arranged in the sidewalls (4, 5) parallel to the back wall (3).

14. Apparatus according to claim 11, **characterized in that** the cover plate (71) is adjustable by means of hooks (73) and eyelets (75) in predetermined positions between the sidewalls (4, 5) and the back wall (3).

## Revendications

1. Dispositif pour conserver et prélever des couches (20), notamment des couches jetables, qui est conformé en forme de récipient cubique présentant une plaque de fond (2), des parois latérales (4, 5) et une paroi arrière (3) et dont la face antérieure est munie d'un élément de recouvrement amovible permettant de libérer du moins partiellement la face antérieure et qui comporte une ouverture de prélèvement (18) prévue à la hauteur de la plaque de fond (2), **caractérisé en ce que** l'élément de recouvrement est formé par au moins une porte (30) fixée mobile en rotation latérale sur la paroi latérale (4, 5) ou par une paroi avant (7) apte à être enlevée par coulissement afin de libérer totalement la face antérieure du récipient cubique lorsque l'on ouvre la porte (30) ou enlève la paroi avant (7) de sorte que l'on peut remplir le récipient ouvert d'une pile de couches par le devant.

2. Dispositif selon la revendication 1, **caractérisé en ce que** des moyens destinés à ouvrir le récipient sont prévus, ces moyens étant formés par des charnières (31), par deux gorges longitudinales (25) dans les parois latérales (4) ou par des zones d'application (43) sur la paroi avant (7) constituée par des pièces d'application (40, 41, 42).

3. Dispositif selon la revendication 1 ou 2, **caractérisé en ce que** l'ouverture de prélèvement (18) est prévue dans la partie basse de l'élément de recouvrement (7 ; 30) et est pratiquée un peu plus haut que la hauteur d'une couche (20) empilée.

4. Dispositif selon la revendication 3, **caractérisé en ce que** l'élément de recouvrement (7 ; 30) est muni d'une fente ou d'une fenêtre de contrôle du niveau de remplissage du récipient.

5. Dispositif selon la revendication 4, **caractérisé en ce que** la largeur de l'élément de recouvrement (30) conformé en forme de porte est inférieure à la largeur de la face antérieure de sorte que l'on obtient, entre la porte (30) et une paroi latérale (4, 5 ; 11), une fente longitudinale faisant office de fente de contrôle (35).

6. Dispositif selon l'une quelconque des revendications 1 à 5, **caractérisé en ce que** des moyens d'adaptation à la taille des couches sont prévus.

7. Dispositif selon la revendication 6, **caractérisé en ce que** ces moyens sont formés par une cloison (16) réglable dans des guides (12) disposés dans les parois latérales (4, 5), parallèlement à la paroi arrière (3).

8. Dispositif selon la revendication 7, **caractérisé en ce qu'**une paroi intermédiaire (11) est disposée entre les deux parois latérales (4, 5) et que des guides (12) sont prévus sur la paroi intermédiaire (11) de part et d'autre de celle-ci, ces guides coïncidant avec les guides (12) sur les parois latérales (4, 5) et servant à introduire par coulissement des cloisons (16) disposées parallèlement à la paroi arrière.

9. Dispositif selon la revendication 1, **caractérisé en ce qu'**il est conformé en deux parties, avec une partie de réception (60) comportant la face antérieure ouverte et avec une partie d'insertion (61) comportant l'élément de recouvrement amovible.

10. Dispositif selon la revendication 9, **caractérisé en ce que** la partie d'insertion (61) est apte à s'encliqueter, par rapport à la partie de réception (6), dans différentes positions correspondant aux tailles des couches.

11. Dispositif selon la revendication 1, **caractérisé en ce que** l'élément de recouvrement amovible est formé par une plaque de recouvrement (40, 41, 42 ; 50, 51, 52 ; 71) apte à être appliquée ou introduite par coulissement.

12. Dispositif selon la revendication 11, **caractérisé en ce que** la plaque de recouvrement est constituée par des éléments d'application (40, 41, 42) angulaires.

13. Dispositif selon la revendication 1, **caractérisé en ce que** l'élément de recouvrement est formé par un élément coulissant (80) qui est réglable dans des positions prédéterminées dans des fentes (83) disposées dans les parois latérales (4, 5), parallèlement à la paroi arrière (3).

14. Dispositif selon la revendication 11, **caractérisé en ce que** la plaque de recouvrement (71) est réglable dans des positions prédéterminées entre les parois latérales (4, 5), parallèlement à la paroi arrière (3), au moyen de crochets (73) et d'oeillets (75).
